(19) Europäisches Patentamt
European Patent Office
Office européen des brevets

(11) Veröffentlichungsnummer: **0 215 357 B1**

(12) **EUROPÄISCHE PATENTSCHRIFT**

(45) Veröffentlichungstag der Patentschrift: **06.05.92**

(51) Int. Cl.[5]: **A61K 31/40**, A61K 31/635, //(A61K31/635,31:40)

(21) Anmeldenummer: **86111973.3**

(22) Anmeldetag: **29.08.86**

(54) **Pharmazeutische Zubereitung zur Behandlung des Bluthochdrucks.**

(30) Priorität: **09.09.85 DE 3532036**

(43) Veröffentlichungstag der Anmeldung:
**25.03.87 Patentblatt 87/13**

(45) Bekanntmachung des Hinweises auf die Patenterteilung:
**06.05.92 Patentblatt 92/19**

(84) Benannte Vertragsstaaten:
**AT BE CH DE FR GB IT LI LU NL SE**

(56) Entgegenhaltungen:
**US-A- 4 217 347**

**CHEMICAL ABSTRACTS, Band 101, 1984, Seite 35, Zusammenfassung Nr. 163417j, Columbus, Ohio, US; P.H. VLASSES et al.: "Comparative antihypertensive effects of enalapril maleate and hydrochlorothiazide, alone and in combination", & J. CLIN. PHARMACOL. 1983, 23(5-6), 227-33**

**PATENT ABSTRACTS OF JAPAN, Band 6, Nr. 24 (C-91)[902], 12. Februar 1982; & JP-A-56 145 220 (DAINIPPON SEIYAKU K.K.) 11-11-1981**

(73) Patentinhaber: **HOECHST AKTIENGESELLSCHAFT**
**Postfach 80 03 20**
**W-6230 Frankfurt am Main 80(DE)**

(72) Erfinder: **Becker, Reinhard, Dr.**
**Adelheidstrasse 101**
**W-6200 Wiesbaden(DE)**
Erfinder: **Geiger, Rolf, Prof.Dr.**
**Heinrich-Bleicher-Strasse 33**
**W-6000 Frankfurt am Main 50(DE)**
Erfinder: **Henning, Rainer, Dr.**
**Rotenhofstrasse 31**
**W-6234 Hattersheim am Main(DE)**
Erfinder: **Teetz, Volker, Dr.**
**An der Tann 20**
**W-6238 Hofheim am Taunus(DE)**
Erfinder: **Urbach, Hansjörg, Dr.**
**Le Lavandoustrasse 41**
**W-6242 Kronberg/Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 215 357 B1

Rank Xerox (UK) Business Services
(3.08/2.18/2.0)

## Beschreibung

Bekanntermaßen kann mit Hemmstoffen des Angiotensin-Converting-Enzyms (ACE-Inhibitoren), wie Captopril oder Enalapril, der Bluthochdruck bei essentiellen Hypertonikern gesenkt werden (Therapiewoche 29 [1979] 7746; Lancet 2 [1981] 543-546). Ein bestimmter Prozentsatz essentieller Hypertoniker spricht jedoch auf solche Stoffe nicht an (Drug Devel. Eval. 4 [1980] 82-91).

Es ist bekannt, daß die antihypertensive Wirkung von Enalapril oder Captopril durch Zusatz diuretisch wirksamer Mengen eines Diuretikums von Thiazid-Typ oder analogen Verbindungen verstärkt wird (Brunner et al., Clin. Exp. Hypertension 2 [1980] 639-657; McGregor et al., Br. Med. J. 284 [1982] 693-696). Als Grund für diesen Effekt wird allgemein angenommen, daß das Diuretikum über einen Salz- und Volumenverlust das Renin-Angiotensin-System stimuliert (P. J. S. Chin et al., J. Pharm. Pharmacol. 37 [1985] 105).

In Arzneim.-Forsch./Drug Res. 34 (II) [1984] 1417-1425 wird über Untersuchungen zur kardiovaskulären Wirkung von 2-[N-[(S)-1-Carboxy-3-phenylpropyl]-L-alanyl]-(1S, 3S, 5S)-2-azabicyclo[3.3.0]octan-3-carbonsäure ("Ramiprilat") berichtet. Dabei wurden Tiere zum Zwecke der Natriumverarmung mehrere Tage mit Furosemid oder Piretanid vorbehandelt.

Wir haben nun überraschenderweise gefunden, daß ACE-Inhibitoren in Kombination mit Schleifendiuretika in niederer Dosierung blutdrucksenkend wirksam sind.

Die Erfindung betrifft daher pharmazeutische Zubereitungen, enthaltend

a) einen Angiotensin-Converting-Enzyme-Inhibitor oder dessen physiologisch verträgliches Salz und

b) ein Schleifendiuretikum oder dessen physiologisch verträgliches Salz.

Zu den Schleifendiuretika im Sinne der vorliegenden Erfindung gehören Furosemid, und Piretanid. Wie aus dem Namen hervorgeht, liegt der Haptangriffspunkt dieser kurz, aber intensiv wirksamen Diuretika an der Henleschen Schleife (vgl. Mutschler, Arzneimittelwirkungen, 4. Auflage, Stuttgart 1981, Seiten 486 und 487). Als solche kommt insbesondere die Verbindung der Formel I in Betracht,

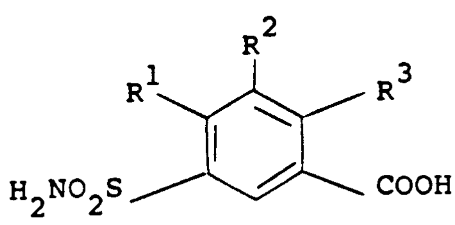

(I)

in welcher

$R^1$ Chlor,

$R^2$ Wasserstoff, und

$R^3$ 2-Furylmethylamino bedeuten.

Geeignet ist ferner Piretanid (s. Formel II).

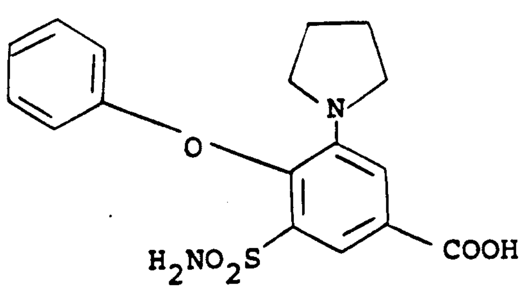

(II)

Geeignete ACE-Inhibitoren sind beispielsweise beschrieben in US-Patent 4 129 571, US-Patent 4 154 960, US-Patent 4 374 829, EP-A-79522, EP-A-79022, EP-A-49658, EP-A-51301, US-Patent 4 454 292, US-Patent 4 374 847, EP-A-72352, US-Patent 4 350 704, EP-A-50800, EP-A-46953, US-Patent andere. Oral wirksame ACE-Inhibitoren sind beispielsweise in Brunner et al., J. Cardiovasc. Pharmacol. 7 (Suppl. I) [1985] S2-S11 beschrieben.

Geeignet sind die aus der EP-A-79022 bekannten ACE-Inhibitoren der Formel III

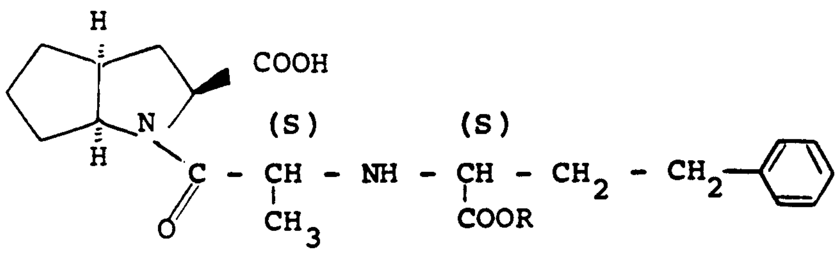

(III)

in welcher
R Wasserstoff, Methyl, Ethyl oder Benzyl
bedeutet,
insbesondere die Verbindung der Formel III, worin R = Ethyl bedeutet (Ramipril).

Weiterhin geeignet sind die aus der EP-A-84164 bekannten ACE-Inhibitoren der Formel IV

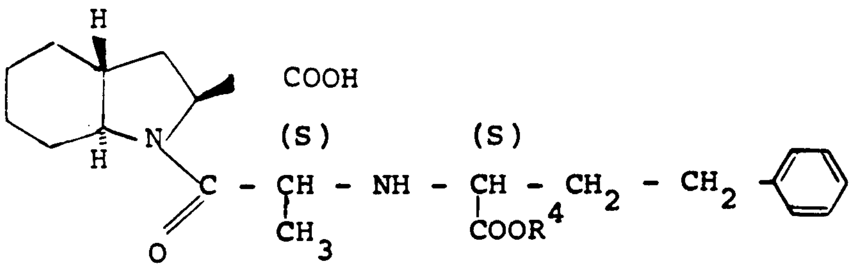

(IV)

in welcher
$R^4$ Wasserstoff, $(C_1\text{-}C_4)$-Alkyl oder Benzyl bedeutet, insbesondere die Verbindung der Formel IV, worin $R^4$ = Ethyl bedeutet.

Bevorzugte erfindungsgemäße Zubereitungen sind somit solche, die eine Verbindung der Formel IV mit $R^4$ = Ethyl zusammen mit Piretanid oder Furosemid enthalten, insbesondere aber solche, die Ramipril zusammen mit Piretanid und die Ramipril zusammen mit Furosemid enthalten.

Die Kombination von ACE-Inhibitoren und Schleifendiuretika wirkt stark und anhaltend blutdrucksenkend und kann daher zur Behandlung von Bluthochdruck verschiedener Genese eingesetzt werden. Von besonderem Interesse ist die Tatsache, daß die Wirkungen der beiden Komponenten sich nicht additiv verhalten; es wird vielmehr ein synergistischer Effekt beobachtet. An der spontan hypertonen Ratte führen in der Kombination bereits Dosen eines ACE-Inhibitors wie Ramipril zu einer Blutdrucksenkung, die allein ohne Wirkung sind, wenn man sie mit Dosen eines Schleifendiuretikums wie Piretanid kombiniert, die alleine keine diuretische Wirkung haben (subdiuretische Dosen). Dies zeigt, daß Schleifendiuretika, insbesondere Verbindungen der o. g. Formel I, das Renin-Angiotensin-System zu stimulieren vermögen, ohne eine diuretische und saluretische Wirkung zu zeigen. Ein solcher Effekt wird mit Verbindungen vom Typ des Hydrochlorothiazids nicht erreicht.

Aus den genannten Gründen ist die erfindungsgemäße Zubereitung den Einzelkomponenten in der Behandlung des Bluthochdrucks überlegen, da sie es erlaubt, geringere Dosierungen der Komponenten zuzuführen, und damit eventuelle toxikologische Probleme zu verringern.

Die Erfindung betrifft auch ein Verfahren zur Herstellung einer solchen Zubereitung, das dadurch gekennzeichnet ist, daß man

a) einen Angiotensin-Converting-Enzyme-Inhibitor der Formel (III) oder (IV) oder dessen physiologisch verträgliches Salz und

b) ein Schleifendiuretikum der Formel (II) oder (II') oder dessen physiologisch verträgliches Salz

zusammen mit physiologisch annehmbaren Trägern und gegebenenfalls weiteren Hilfs- oder Zusatzstoffen in eine geeignete Darreichungsform bringt.

Die Erfindung betrifft weiterhin ganz allgemein Erzeugnisse, enthaltend

a) einen Angiotensin-Converting-Enzyme-Inhibitor der Formel (III) oder (IV) oder dessen physiologisch verträgliches Salz und

b) ein Schleifendiuretikum der Formel (II) oder (II') oder dessen physiologisch verträgliches Salz in einer subdiuretischen Dosis,

als Kombinationspräparat zur gleichzeitigen, getrennten oder zeitlich abgestuften Anwendung bei der Behandlung des Bluthochdrucks.

Das Gewichts-Verhältnis ACE-Inhibitor: Schleifendiuretikum in den genannten Zubereitungen und Erzeugnissen bewegt sich, je nach Aktivität der Wirkstoffe, vorzugsweise zwischen 10 : 1 und 1 : 500. Für Ramipril (=A) + Piretanid (=B) z.B. bewegt sich A : B vorzugsweise zwischen 4 : 1 und 1 : 10, insbesondere zwischen 2 : 1 und 1 : 3. Demgegenüber ist bei Ramipril (=A) + Furosemid (=C) das Verhältnis A : C bevorzugt 1 : 1 bis 1 : 200, insbesondere 1 : 4 bis 1 : 40.

Die Schleifendiuretika der Formeln (II) und (II') bilden auf Grund ihres $pK_a$-Wertes (pKa von Furosemid: 3,8) Salze mit ACE-Inhibitoren der Formeln (III) und (IV), wobei die Verbindung der Formel (II) und (II') in ihr Kation übergeht unter Protonierung der NH-Funktion in die Verbindung der Formel (III) und (IV). Falls die Verbindung der Formel (III) oder (IV) (R = H) als Zwitterion vorliegt, wird eine Carboxylat-Funktion protoniert.

Die Erfindung betrifft daher auch ein Salz eines Schleifendiuretikums der Formel (II) oder (II') mit einem ACE-Inhibitor sowie pharmazeutische Zubereitungen und Erzeugnisse, die ein solches Salz enthalten. Falls erforderlich können die genannten Zubereitungen und Erzeugnisse zusätzlich ein Schleifendiuretikum der Formel (II) oder (II') in freier Form oder dessen physiologisch verträgliches Salz oder einen ACE-Inhibitor in freier Form oder dessen physiologisch verträgliches Salz enthalten.

Bevorzugt sind Salze von Verbindungen der Formel (II) oder (II') mit Verbindungen der Formel III oder IV, insbesondere solche von Piretanid oder Furosemid mit Ramipril oder einer Verbindung der Formel IV mit $R^4$ = Ethyl.

Man stellt die Salze her, indem man stöchiometrische Mengen der Reaktionspartner in einem geeigneten Lösungsmittel auflöst und durch Einengen, Abkühlen oder Zugabe eines weiteren Lösungsmittels, in dem die Salze weniger löslich sind, diese in fester Form abscheidet. Die Salze können in der oben beschriebenen Weise zu Zubereitungen oder Erzeugnissen verarbeitet werden.

Die Dosen des ACE-Inhibitors und des Schleifendiuretikums in den erfindungsgemäßen Zubereitungen bzw. Erzeugnissen werden vorzugsweise jeweils so gewählt, daß der ACE-Inhibitor und/oder das Schleifendiuretikum für sich allein noch keine oder keine volle Wirkung zeigen würde(n). So genügt bei den Schleifendiuretika schon eine Dosis, die weit unterhalb des $ED_{50}$-Werts, etwa bei seiner diuretischen Schwellendosis liegt. ACE-Inhibitoren als Komponenten können bereits in Dosen eingesetzt werden, die etwa bei der minimalen, für eine Plasma-ACE-Hemmung ausreichenden Dosis liegen (Bestimmung siehe: Metzger et al., Arzneim.-Forsch./Drug Res. 34 (II), 1402, 1403); sie können damit unterhalb solcher liegen, die für eine akut blutdrucksenkende Wirkung bei Anwendung eines ACE-Hemmers allein erforderlich sind.

Bei der erfindungsgemäßen Anwendung bei Säugern, vorzugsweise beim Menschen, bewegen sich beispielsweise die Dosen eines ACE-Inhibitors der o. g. Formel III bzw. IV im Bereich von 0,05 bis 2 mg/kg/Tag und die eines Schleifendiuretikums im Bereich von 0,2 bis 25 mg/kg/Tag.

Die erfindungsgemäßen Zubereitungen bzw. Erzeugnisse können parenteral oder oral verabreicht werden. Bevorzugt ist die orale Applikationsform.

Die pharmakologisch verwendbaren Kombinationen der vorliegenden Erfindung und ihre Salze können zur Herstellung von pharmazeutischen Präparaten verwendet werden, welche eine wirksame Menge der Aktivsubstanzen zusammen mit Trägerstoffen enthalten und die sich zur enteralen und parenteralen Verabreichung eignen. Vorzugsweise verwendet werden Tabletten oder Gelatinekapseln, welche die Wirkstoffe zusammen mit Verdünnungsmitteln, z.B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganth, Methylcellulose, Natriumcarboxymethylcellulose und/oder Polyvinylpyrrolidon und, falls benötigt, Farbstoff, Geschmacksstoffe und Süßmittel.

Injizierbare Lösungen sind vorzugsweise isotonische wäßrige Lösungen oder Suspensionen, die sterilisiert sein können und Hilfsstoffe wie Konservier-, Stabilisierungs-, Netz-und/oder Emulgiermittel, Löslichkeitsvermitteler, Salze zur Regulierung des osmotischen Drucks und/oder Puffersubstanzen enthalten können.

Die erfindungsgemäßen pharmazeutischen Präparate, die, wenn erwünscht, weitere pharmakologisch wertvolle Stoffe enthalten können, werden z.B. mittels konventioneller Misch-, Granulier- und Dragierverfahren, hergestellt und enthalten 0,1 % bis etwa 75 %, bevorzugt etwa 1 % bis etwa 50 % der Wirkstoffe.

Dabei werden die Wirkstoffe zusammen mit den oben genannten Hilfs- und Zusatzstoffen in einer Mischvorrichtung bei 5 - 50 °C vermischt oder gelöst und dann beispielsweise zu Tabletten verpreßt oder in Gelatinekapseln oder Ampullen abgefüllt.

Die folgenden Beispiele dienen zur Erläuterung der vorliegenden Erfindung, ohne daß diese darauf beschränkt wäre.

Beispiel 1

Wirkung der Kombination von Ramipril ( = A) und Piretanid ( = B) an der spontan hypertensiven Ratte

10 spontan hypertensive Ratten (Wistar-Kyoto) wurden instrumentiert und während der Versuche in Stoffwechselkäfigen gehalten. Ramipril ( = A) (1 mg/kg) und Piretanid ( = B) (1,2 und 16 mg/kg) werden in Tylose oral mit Magensonde verabreicht. Die Kontrollgruppe erhielt nur Tylose. Die ausgeschiedene Urinmenge nach 5 Stunden, die Natriumausscheidung nach 5 Stunden sowie der arterielle Mitteldruck wurden bestimmt.

Tabelle 1

| Urinausscheidung über 5 Stunden | |
|---|---|
| | Urinmenge (µ1/100g.5h) |
| Tylose (1 ml/kg) | 460±65 |
| A (1 mg/kg) | 760±185 |
| B (1 mg/kg) | 900±180 |
| B (2 mg/kg) | 1470±140 |
| B (16 mg/kg) | 3520±405 |
| A (1 mg/kg) + B (1 mg/kg) | 1100±145 |
| A (1 mg/kg) + B (2 mg/kg) | 1545±130 |
| A (1 mg/kg) + B (16 mg/kg) | 3590±285 |

Tabelle 2

| Natriumausscheidung über 5 Stunden | |
|---|---|
| | $m_{Na}(\bar{x}$±SD±SEM) (mmol/150g.5h) |
| Tylose (1 ml/kg) | 0,021±0,0009±0,003 |
| A (1 mg/kg) | 0,080±0,049 ±0,016 |
| B (1 mg/kg) | 0,082±0,057±0,021 |
| B (2 mg/kg) | 0,156±0,063±0,022 |
| B (16 mg/kg) | 0,462±0,146±0,052 |
| A (1 mg/kg) + B (1 mg/kg) | 0,157±0,050±0,321 |
| A (1 mg/kg) + B (2 mg/kg) | 0,158±0,033±0,012 |
| A (1 mg/kg) + B (16 mg/kg) | 0,513±0,076±0,031 |

In der Abbildung ist der zeitliche Verlauf des mittleren Blutdrucks ($MBP_t$) in % des Ausgangsblutdrucks ($MBP_0$) dargestellt. Dabei haben die einzelnen Kurven die folgenden Bedeutungen:

| | |
|---|---|
| Tylose (1 mg/kg): | Kurve h |
| A (1 mg/kg): | Kurve a |
| B (1 mg/kg): | Kurve b |
| B (2 mg/kg): | Kurve c |
| B (16 mg/kg): | Kurve d |
| A (1 mg/kg) + B (1 mg/kg) : | Kurve g |
| A (1 mg/kg + B (2 mg/kg) : | Kurve f |
| A (1 mg/kg) + B (16 mg/kg) : | Kurve e |

Beispiel 2

Herstellung eines oralen Kombinationspräparats aus Ramipril ( = A) und Piretanid ( = B)

1000 Tabletten, die je 1 mg A und B enthalten, werden mit den folgenden Hilfsmitteln hergestellt:

| | |
|---|---|
| A | 1 g |
| B | 1 g |
| Maisstärke | 140 g |
| Gelatine | 7,5 g |
| Mikrokristalline Cellulose | 2,5 g |
| Magnesiumstearat | 2,5 g |

A und B werden mit einer wäßrigen Gelatine-Lösung gemischt. Die Mischung wird getrocknet und zu einem Granulat vermahlen. Mikrokristalline Cellulose und Magnesiumstearat sowie Maisstärke werden mit dem Granulat vermischt. Das entstehende Granulat wird zu 1000 Tabletten gepreßt, wobei jede Tablette je 1 mg A und B enthält.

Beispiel 3

Herstellung eines parenteralen Kombinationspräparots aus Ramipril ( = A) und Piretanid ( = B)

Die Herstellung einer Injektionslösung zur Behandlung der Hypertonie wird im folgenden beschrieben:

| | |
|---|---|
| A | 0,25 g |
| B | 0,25 g |
| Methylparaben | 5 g |
| Propylparaben | 1 g |
| Natriumchlorid | 25 g |
| Wasser für Injektion | 5 l |

A, B, die Konservierungsstoffe und Natriumchlorid werden in Wasser für Injektion gelöst und mit Wasser für Injektion auf 5 l aufgefüllt. Die Lösung wird steril gefiltert und aseptisch in vorsterilisierte Flaschen gefüllt die mit sterilisierten Gummikappen verschlossen werden. Jede Flasche enthält 5 ml Lösung.

Beispiel 4

Herstellung eines oralen Kombinationspräparats aus Ramipril ( = A) und Furosemid ( = C)

1000 Tabletten, die 5 mg A und 20 mg C enthalten, wurden mit den folgenden Hilfsmitteln hergestellt:

| | |
|---|---|
| A | 5 g |
| C | 20 g |
| Maisstärke | 140 g |
| Gelatine | 7,5 g |
| Mikrokristalline Cellulose | 2,5 g |
| Magnesiumstearat | 2,5 g |

A und C werden mit einer wäßrigen Gelatine-Lösung gemischt. Die Mischung wird getrocknet und zu einem Granulat vermahlen. Mikrokristalline Cellulose und Magnesiumstearat sowie Maisstärke werden mit dem Granulat vermischt. Das entstehende Granulat wird zu 1000 Tabletten gepreßt, wobei jede Tablette 5 mg A und 20 mg C enthält.

Beispiel 5

In Analogie zu Beispiel 2 werden Tabletten hergestellt, die pro Tablette 4 mg Ramipril und 1 mg Piretanid enthalten.

Beispiel 6

Zu Analogie zu Beispiel 2 werden Tabletten hergestellt, die pro Tablette 0,5 mg Ramipril und 5 mg Piretanid enthalten.

Beispiel 7

In Analogie zu Beispiel 4 werden Tabletten hergestellt, die pro Tablette 25 mg Ramipril und 30 mg Furosemid enthalten.

Beispiel 8

In Analogie zu Beispiel 4 werden Tabletten hergestellt, die 1 mg Ramipril und 25 mg Furosemid pro Tablette enthalten.

## Patentansprüche

### Patentansprüche für folgende Vertragsstaaten : BE, CH, DE, FR, GB, IT, LI, LU, NL, SE

**1.** Pharmazeutische Zubereitung, enthaltend
a) einen Angiotensin-Converting-Enzyme-Inhibitor oder dessen Physiologisch verträgliches Salz und
b) ein Schleifendiuretikum oder dessen physiologisch verträgliches Salz, dadurch gekennzeichnet, daß sie das Schleifendiuretikum der Formel (II) oder (II') in einer subdiuretischen Dosis enthält,

(II)

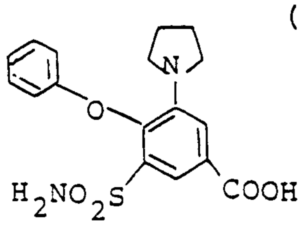

(II')

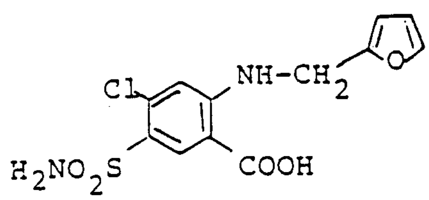

und daß sie einen Angiotensin-Converting Enzyme Inhibitor der Formel (III) oder (IV) enthält

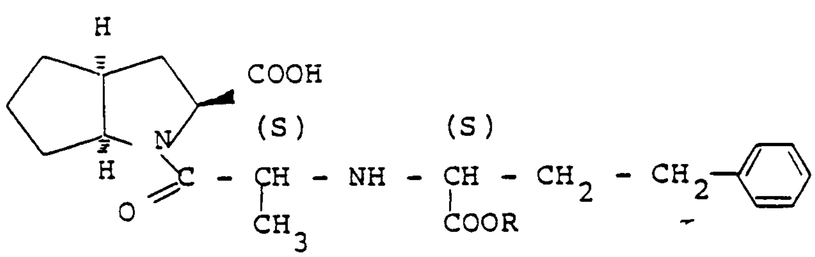

(III)

in welcher
R Wasserstoff, Mehtyl, Ethyl oder Benzyl
bedeutet
oder

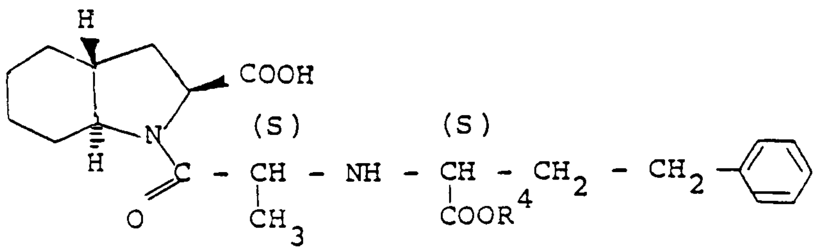

(IV)

in welcher
$R^4$ Wasserstoff, $(C_1\text{-}C_4)$-Alkyl oder Benzyl bedeutet.

2. Zubereitung gemäß Anspruch 1, dadurch gekennzeichnet, daß der Angiotensin-Converting-Enzyme-Inhibitor die Formel (III) oder (IV), in welcher R bzw. $R^4$ Ethyl bedeuteten, aufweist.

3. Verfahren zur Herstellung einer Zubereitung gemäß einem der Ansprüche 1-2 dadurch gekennzeichnet, daß man
   a) einen Angiotensin-Converting-Enzyme-Inhibitor oder dessen Salz und
   b) ein Schleifendiuretikum oder dessen physiologisch verträgliches Salz

   zusammen mit physiologisch annehmbaren Träger und gegebenenfalls weiteren Hilfs- oder Zusatzstoffen in eine geeignete Darreichungsform bringt.

4. Zubereitung gemäß einem der Ansprüche 1-2 zur Anwendung als Heilmittel bei der Behandlung des Bluthochdrucks.

5. Erzeugnis, enthaltend
   a) einen Angiotensin-Converting-Enzyme-Inhibitor oder dessen physiologisch verträglichen Salz und
   b) ein Schleifendiuretikum oder dessen physiologisch verträgliches Salz

   als Kombinationspräparat zur gleichzeitigen, getrennten oder zeitlich abgestuften Anwendung bei der Behandlung des Bluthochdrucks, dadurch gekennzeichnet, daß seine Komponenten wie in Anspruch 1 definiert sind.

**Patentansprüche für folgenden Vertragsstaat : AT**

1. Verfahren zur Herstellung einer Pharmazeutischen Zubereitung, enthaltend
   a) einen Angiotensin-Converting-Enzyme-Inhibitor oder dessen Physiologisch verträglisches Salz und
   b) ein Schleifendiuretikum oder dessen physiologisch verträgliches Salz, dadurch gekennzeichnet, daß man ein Schleifendiuretikum der Formel (II) oder (II') in einer subdiuretischen Dosis

(II)

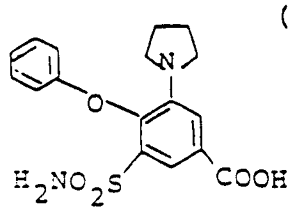

(II')

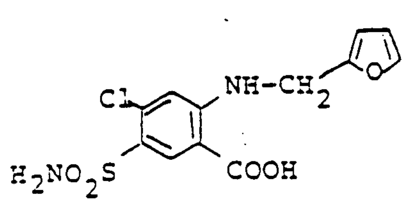

und einen
Angiotensin-Converting Enzyme Inhibitor der Formel (III) oder (IV)

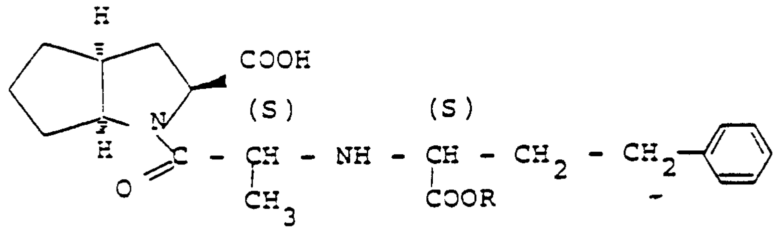
(III)

in welcher
R Wasserstoff, Mehtyl, Ethyl oder Benzyl
bedeutet
oder

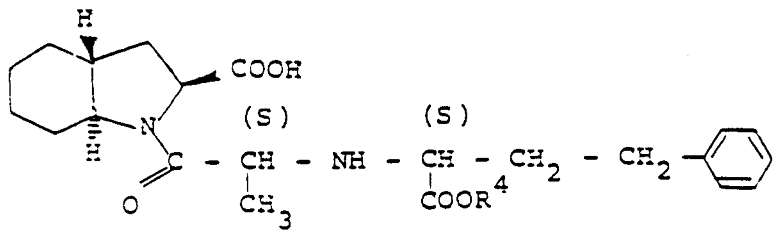
(IV)

in welcher $R^4$ Wasserstoff, $(C_1\text{-}C_4)$-Alkyl oder Benzyl bedeutet,
zusammen mit einem Physiologisch annehmbaren Träger und gegebenenfalls weiteren Hilfs- oder Zusatzstoffen in eine Geeignete Darreichungsform bringt.

**2.** Verfahren zur Herstellung einer Zubereitung gemäß Anspruch 1 dadurch gekennzeichnet, daß der Angiotensin-Converting-Enzyme-Inhibitor die Formel (III) oder (IV), in welcher R bzw. $R^4$ Ethyl bedeuteten, aufweist.

**3.** Verfahren zur Herstellung eines Erzeugnisses, enthaltend
a) einen Angiotensin-Converting-Enzyme-Inhibitor oder dessen verträgliches Salz und
b) ein Schleifendiuretikum oder dessen physiologisch verträgliches Salz
als Kombinationspräparat zur gleichzeitigen, getrennten oder zeitlich abgestuften Anwendung bei der Behandlung des Bluthochdrucks,
dadurch gekennzeichnet, daß man die beiden wie in Anspruch 1 definierten Komponenten jeweils zusammen mit einem physiologisch annehmbaren Träger und gegebenenfalls weiteren Hilfs- oder Zusatzstoffen in eine geeignete Darreichungsform bringt.

**Claims**

**Claims for the following Contracting States : BE, CH, DE, FR, GB, IT, LI, LU, NL, SE**

**1.** A pharmaceutical composition containing
a) an angiotensin converting enzyme inhibitor or its physiologically tolerated salt and

b) a loop diuretic or its physiologically tolerated salt, which contains the loop diuretic of the formula (II) or (II') in a subdiuretic dose,

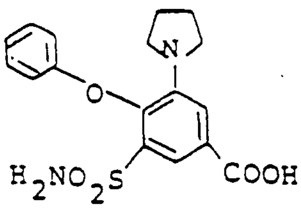

(II)

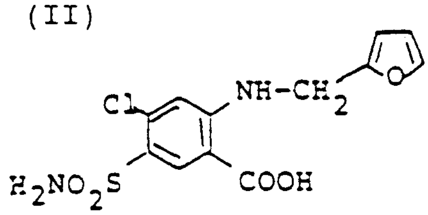

(II')

and contains an angiotensin converting enzyme inhibitor of the formula (III) or (IV)

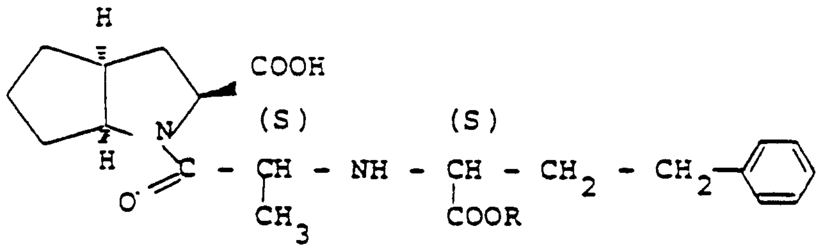

(III)

in which
R denotes hydrogen, methyl, ethyl or benzyl,
or

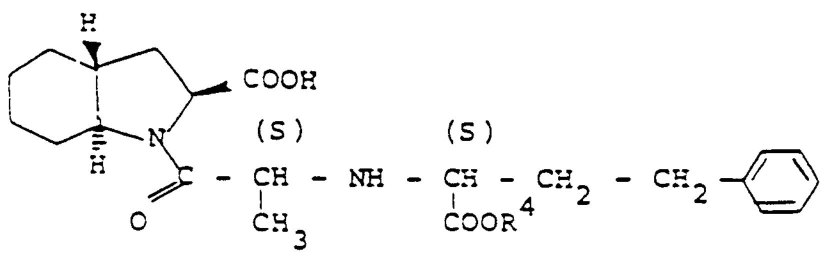

(IV)

in which
$R^4$ denotes hydrogen, $(C_1\text{-}C_4)$-alkyl or benzyl.

**2.** A composition as claimed in claim 1, wherein the angiotensin converting enzyme inhibitor has the formula (III) or (IV) in which R or $R^4$ denotes ethyl.

**3.** A process for the preparation of a composition as claimed in one of claims 1-2, which comprises conversion into a suitable form for administration of
a) an angiotensin converting enzyme inhibitor or its salt and
b) a loop diuretic or its physiologically tolerated salt
together with physiologically acceptable vehicles and, where appropriate, other auxiliaries or additives.

**4.** A composition as claimed in one of claims 1-2 for use as a medicine for the treatment of high blood pressure.

**5.** A product containing
a) an angiotensin converting enzyme inhibitor or its physiologically tolerated salt and

b) a loop diuretic or its physiologically tolerated salt

as combination product for concurrent, separate or sequential administration for the treatment of high blood pressure, whose components are defined as in claim 1.

**Claims for the following Contracting State : AT**

**1.** A process for the preparation of a pharmaceutical composition containing

a) an angiotensin converting enzyme inhibitor or its physiologically tolerated salt, and

b) a loop diuretic or its physiologically tolerated salt, which comprises conversion into a suitable form for administration of a loop diuretic of the formula (II) or (II') in a subdiuretic dose

(II)

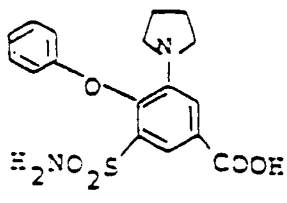

(II')

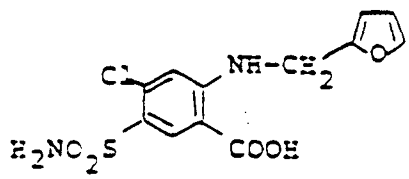

and an angiotensin converting enzyme inhibitor of the formula (III) or (IV)

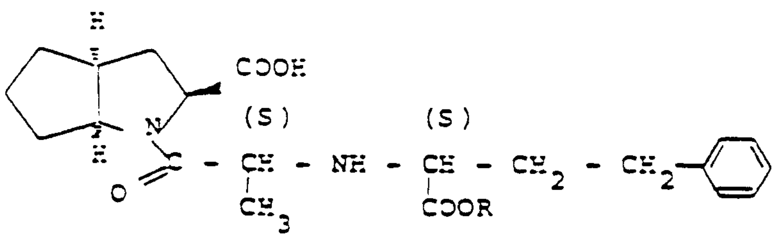

(III)

in which

R denotes hydrogen, methyl, ethyl or benzyl,

or

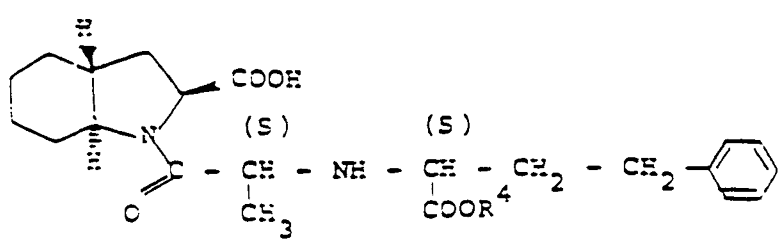

(IV)

in which

$R^4$ denotes hydrogen, $(C_1-C_4)$-alkyl or benzyl, together with a physiologically acceptable vehicle and, where appropriate, other auxiliaries or additives.

**2.** The process for the preparation of a composition as claimed in claim 1, wherein the angiotensin converting enzyme inhibitor has the formula (III) or (IV) in which R or $R^4$ denotes ethyl.

**3.** A process for the preparation of a product containing

a) an angiotensin converting enzyme inhibitor or its physiologically tolerated salt, and

b) a loop diuretic or its physiologically tolerated salt
as combination product for concurrent, separate or sequential administration for the treatment of high blood pressure, which comprises conversion into a suitable form for administration of the two components as defined in claim 1, in each case together with a physiologically acceptable vehicle and, where appropriate, other auxiliaries or additives.

## Revendications

**Revendications pour les Etats contractants suivants : BE, CH, DE, FR, GB, IT, LI, LU, NL, SE**

**1.** Préparation pharmaceutique, contenant:

a) un inhibiteur d'enzyme de conversion de l'angiotensine ou un de ses sels physiologiquement compatibles, et

b) un diurétique de l'anse ou un de ses sels physiologiquement compatibles,

caractérisée en ce qu'elle renferme le diurétique de l'anse, de formule (II) ou (II'), à une dose sous-diurétique,

(II)

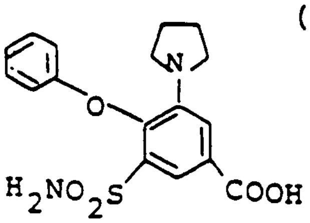

(II')

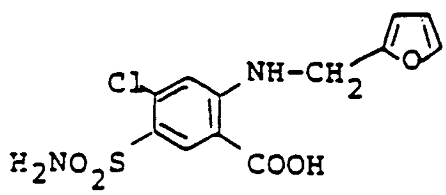

et qu'elle renferme un inhibiteur d'enzyme de conversion de l'angiotensine de formule (III) ou (IV)

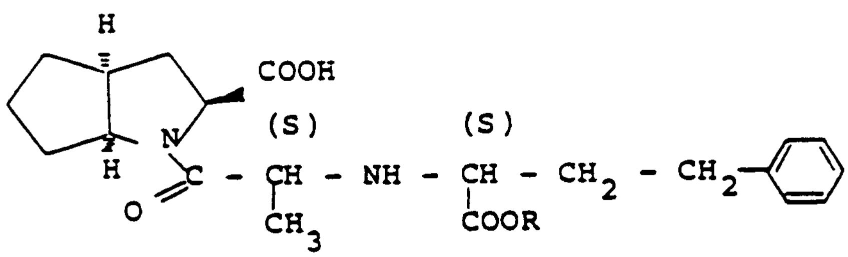

(III)

dans laquelle
R est hydrogène, méthyle, éthyle ou benzyle,
ou

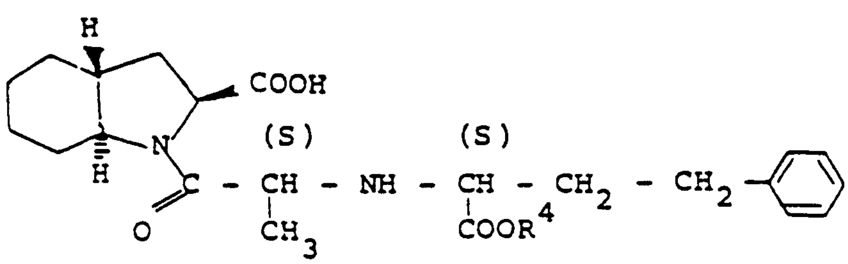

(IV)

dans laquelle
$R^4$ représente un atome d'hydrogène ou un groupe alkyle en $C_{1-4}$ ou benzyle.

**2.** Préparation selon la revendication 1, caractérisée en ce que l'inhibiteur d'enzyme de conversion de l'angiotensine est représenté par la formule (III) ou (IV), dans laquelle R ou $R^4$ est éthyle.

**3.** Procédé de fabrication d'une préparation selon l'une des revendications 1 ou 2, caractérisé en ce qu'on met sous une forme d'administration appropriée

a) un inhibiteur d'enzyme de conversion de l'angiotensine ou un de ses sels physiologiquement compatibles, et

b) un diurétique de l'anse ou un de ses sels physiologiquement compatibles,

avec des véhicules physiologiquement acceptables et éventuellement d'autres excipients ou additifs.

**4.** Préparation selon une des revendications 1 ou 2, pour son utilisation comme médicament dans le traitement de l'hypertension.

**5.** Produit, contenant

a) un inhibiteur d'enzyme de conversion de l'angiotensine ou un de ses sels physiologiquement compatibles, et

b) un diurétique de l'anse ou un de ses sels physiologiquement compatibles,

comme préparation combinée pour l'administration simultanée, séparée ou répartie dans le temps, dans le traitement de l'hypertension, caractérisé en ce que ses composants sont définis comme dans la revendication 1.

**Revendications pour l'Etat contractant suivant : AT**

**1.** Procédé de fabrication d'une préparation pharmaceutique, contenant:

a) un inhibiteur d'enzyme de conversion de l'angiotensine ou un de ses sels physiologiquement compatibles, et

b) un diurétique de l'anse ou un de ses sels physiologiquement compatibles, caractérisé en ce qu'on met sous une forme d'administration appropriée un diurétique de l'anse, de formule (II) ou (II'), à une dose sous-diurétique,

(II)

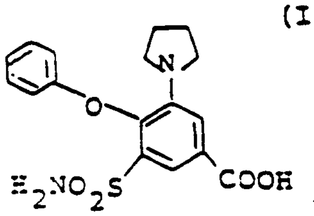

(II')

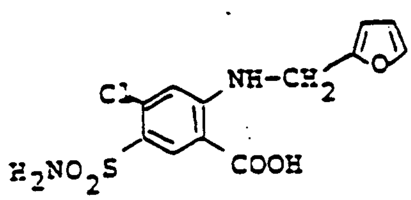

et un inhibiteur d'enzyme de conversion de l'angiotensine de formule (III) ou (IV)

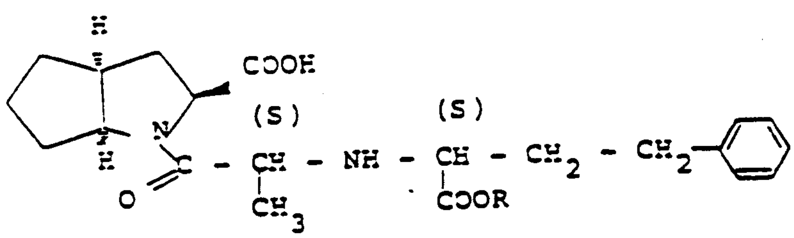

(III)

dans laquelle

R est hydrogène, méthyle, éthyle ou benzyle,

ou

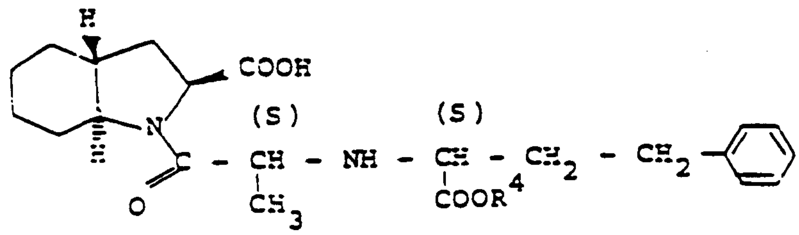

(IV)

dans laquelle
$R^4$ représente un atome d'hydrogène ou un groupe alkyle en $C_{1-4}$ ou benzyle, conjointement avec un véhicule physiologiquement acceptable et éventuellement d'autres excipients ou additifs.

**2.** Procédé de fabrication d'une préparation selon la revendication 1, caractérisé en ce que l'inhibiteur d'enzyme de conversion de l'angiotensine est représenté par la formule (III) ou (IV), dans laquelle R ou $R^4$ est éthyle.

**3.** Procédé de fabrication d'un produit, contenant
a) un inhibiteur d'enzyme de conversion de l'angiotensine ou un de ses sels physiologiquement compatibles, et
b) un diurétique de l'anse ou un de ses sels physiologiquement compatibles,
comme préparation combinée pour l'administration simultanée, séparée ou répartie dans le temps, dans le traitement de l'hypertension, caractérisé en ce qu'on met sous une forme d'administration appropriée les deux composants définis dans la revendication 1, en même temps, chaque fois, qu'un véhicule physiologiquement compatible et éventuellement d'autres excipients ou additifs.

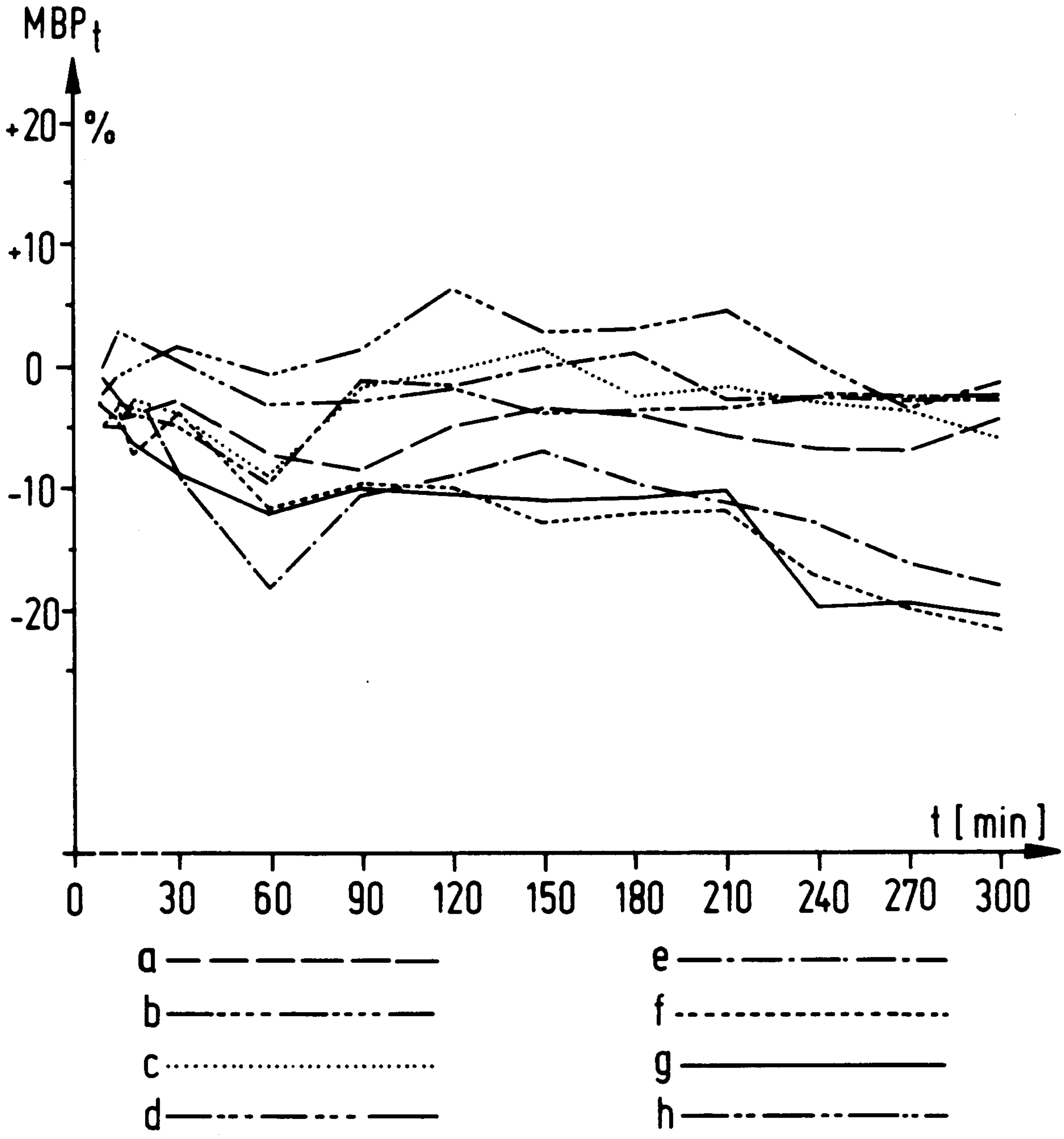
MBPt
%
+20
+10
0
-10
-20
t [min]
0
30
60
90
120
150
180
210
240
270
300
a
b
c
d
e
f
g
h